# EUROPÄISCHE PATENTSCHRIFT

(11) **EP 0 820 785 B1**
(45) Veröffentlichungstag und Bekanntmachung des Hinweises auf die Patenterteilung: **05.06.2002**
(21) Anmeldenummer: 96112189.4
(22) Anmeldetag: 27.07.1996
(51) Int. Cl.: A61M 27/00

(54) **Anordnung mit einer Ureterschiene und einem damit über eine Verschraubung verbindbaren Mandrin, sowie einer Hilfsschiene**
Ureteral conduit assembly with screw fit interconnection between tube, mandrel and auxiliary tube
Assemblage d'un conduit urétéral avec accouplement entre tube, mandrin et tube auxiliaire

(43) Veröffentlichungstag der Anmeldung: 28.01.1998
(73) Patentinhaber: Sachse, Hans, Professor Dr., 90425 Nürnberg (DE)
(72) Erfinder: Sachse, Hans, Professor Dr., 90425 Nürnberg (DE)
(74) Vertreter: Marsh, Roy David

(56) Entgegenhaltungen:
- DE-A- 3 824 244

## Beschreibung

Die Erfindung geht aus von einer Anordnung mit einer Ureterschiene, einem Mandrin und einer Hilfsschiene, wobei eine lösbare Gewindeverbindung zwischen dem Mandrin und der hohlen Ureterschiene vorgesehen ist, welche die Ureterschiene auf Drehmitnahme mit dem Mandrin verbindet, wobei nach Lösen dieser Gewindeverbindung der Mandrin in Längsrichtung der Ureterschiene zu dieser verschiebbar und ganz herausziehbar ist und wobei des Mandrin mittels einer von Hand zu betätigenden Vorrichtung zur Ureterschiene und Hilfsschiene um die gemeinsame Längsachse vesdiehbar ist (Oberbegriff des Anspruches 1). Eine derartige Anordnung ist aus DE-OS 38 24 244 bekannt, wobei ineinandergreifende Gewinde an der Ureterschiene und am Mandrin vorgesehen sind. Dabei erfolgte das Lösen dieser Schraubverbindung zwischen Mandrin und Ureterschiene durch eine entsprechende Drehung des Mandrins relativ zur Ureterschiene. Da sich hierbei praktisch die gesamte Ureterschiene im Körper des Patienten befindet wird durch den Eingriff von Ausladungen der Hilfsschiene in entsprechende Aussparungen der Ureterschiene das Mitdrehen der Ureterschiene verhindert. Für das Verdrehen des Mandrins war an seinem arztseitigen, aus dem Körper des Patienten vorragenden Ende ein Handgriff vorgesehen. Ferner war zur Fixierung der Lage von Ureterschiene, Hilfsschiene und Mandrin zueinander eine von außen auf der Hilfsschiene in deren arztseitigen Endbereich anzusetzende Klemmverbindung erforderlich. Eine solche Klemmverbindung ist entsprechend aufwendig und bedingt in der Handhabung die Anbringung eines solchen gesonderten Bauteiles an der Anordnung. Sie stört bei der Handhabung auch aufgrund ihres Gewichtes.

Die Aufgaben- bzw. Problemstellung der Erfindung besteht demgegenüber darin, eine Anordnung gemäß dem Oberbegriff des Anspruches 1 so auszugestalten, daß unter Vermeidung der o.g. Klemmverbindung vom Arzt während des Einbringens der Ureterschiene in den Ureter die erforderlichen Drehbewegungen der Anordnung um ihre Längsachse und auch in beiden Richtungen ihrer Längsachse einwandfrei durchführbar sind, wobei die hierzu erforderlichen konstruktiven Mittel gegenüber dem zuvor erläuterten Stand der Technik in Konstruktion und Handhabung vorteilhafter sind.

Zur Lösung dieser Aufgaben- bzw. Problemstellung wird, ausgehend vom eingangs genannten Oberbegriff des Anspruches 1, gemäß dem Kennzeichen des Anspruches 1 zunächst vorgesehen, daß der Mandrin mit der Hilfsschiene in einer Gewindeverbindung steht, die eine Drehmitnahme in beiden Drehrichtungen zwischen diesen beiden Teilen bildet und sich bei in den Ureter eingeführter Ureterschiene außerhalb des Körpers des Patienten befindet. Hiermit ist es während des Einbringens der Anordnung in den Körper bis zur Positionierung der Ureterschiene im Ureter nicht notwendig, für die bisher gebräuchliche, gesonderte Klemmverbindung zwischen Mandrin und Hilfsschiene vorzusehen und zu handhaben, da während dieses Einbringens die Gewindeverbindung zwischen Mandrin und Hilfsschiene so viel an Reibungswiderstand hat, daß sie dabei Verdrehungen am Mandrin oder an der Hilfsschiene auf den jeweils anderen Teil mit überträgt. Nachdem die Ureterschiene in den Ureter eingebracht ist und somit das Nierenkelchbecken mit der Blase verbindet, wird durch ein Drehen des Mandrins relativ zur Hilfsschiene erreicht, daß die Gewindeverbindung zwischen Mandrin und Ureterschiene herausgeschraubt und damit der Mandrin von der Ureterschiene gelöst wird. Danach können der Mandrin und die Hilfsschiene entfernt werden. Das gleiche gilt für einen in der Regel vorgesehenen Steuerungsdraht, der sich von der Arztseite der Anordnung her durch einen inneren Hohlraum eines hierzu vorgesehenen hohlen Mandrins und durch das Lumen der Ureterschiene hindurch bis in deren Spitzenbereich erstreckt. Gegenüber den o.g. Hilfsmitteln in Form von Klemmen zwischen Hilfsschiene und Mandrin ist die Anordnung nach der Erfindung zum einen baulich einfacher und dadurch mit geringeren Kosten herzustellen. Außerdem erleichtert sie die Handhabung durch den Arzt. Die Behandlungsdauer wird verkürzt. Ihr Gewicht ist wesentlich leichter als die vorbekannte Klemmverbindung. Auch dies erleichtert die Handhabung. Die Anordnung nach der Erfindung kann ab Fabrik im sofort gebrauchsfertigen Zustand geliefert werden, in dem sich das Außengewinde der Mandrinspitze im patientenseitigen Innenbereich der Ureterschiene befindet. Diese Vormontage erlaubt eine wesentliche Reduzierung der Verpackungslänge. Die Ureterschiene und der übrige Teil der Anordnung liegen in der Verpackung nebeneinander. Dies erlaubt eine leichtere Stapelung in der Klinik und eine Reduzierung der Herstellungskosten der Verpackung.

Eine bevorzugte Ausführung der Erfindung ist Gegenstand des Anspruches 4. Hiermit wird sowohl das zuvor beschriebene gemeinsame Verdrehen von Mandrin und Hilfsschiene um ihre gemeinsame Längsachse ermöglicht, als auch ein Verdrehen der Hilfsschiene relativ zum Mandrin um die gemeinsame Längsachse. Hierzu ist lediglich die Hilfsschiene mit einer Hand festzuhalten und der Mandrin mit der anderen Hand zu verdrehen. Hierzu können am Mandrin und an der Hilfsschiene Handhaben gemäß den Ansprüchen 5 bis 7 vorgesehen sein.

Eine weitere, bevorzugte Ausführung der Erfindung ist Gegenstand des Anspruches 12. Durch das Anstoßen des patientenseitigen Stirnendes der Hilfsschiene am arztseitigen Stirnende der Ureterschiene wird eine zusätzliche Kraft für das Ablösen des patientenseitigen Endes des Mandrins aus der Ureterschiene dadurch erreicht, daß beim Verdrehen des Mandrins relativ zur Hilfsschiene der Gewindeeingriff zwischen diesen beiden Teilen eine entsprechende Längsverschiebung des Mandrins zur Hilfsschiene und damit zur Ureterschiene bewirkt, wobei eine gleichzeitige Drehung des Mandrins relativ zur Ureterschiene um die gemeinsame Längsachse erfolgt.

Die Merkmale des Anspruches 16 erleichtern das Herausschrauben des Mandrins aus der Ureterschiene.

Weitere Vorteile und Merkmale der Erfindung sind den weiteren Ansprüchen, auf deren Inhalt hiermit ausdrücklich Bezug genommen wird, der nachfolgenden Beschreibung und der zugehörigen Zeichnung von erfindungsgemäßen Ausführungsmöglichkeiten zu entnehmen. In der Zeichnung zeigt:
- Fig. 1:: eine Ausführungsmöglichkeit der Erfindung im Längsschnitt,
- Fig. 2:: die Einzelheit A der Fig. 1 im vergrößerten Maßstab,
- Fig. 2a:: im Längsschnitt eine Abwandlung der Gewindeverbindung gemäß Fig. 2,
- Fig. 3:: in einem gegenüber Fig. 1 vergrößerten Maßstab eine weitere Ausführungsmöglichkeit der Erfindung für die Gewindeverbindung zwischen Mandrin und Hilfsschiene.

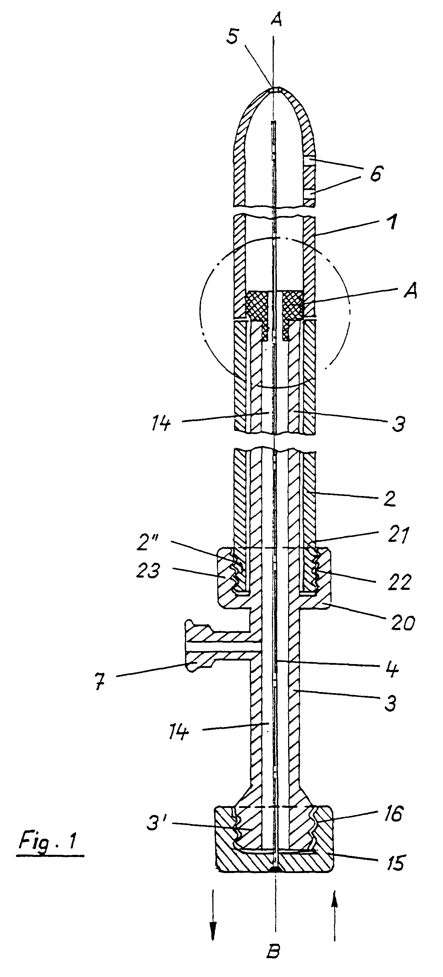

Fig. 1 zeigt die Bauteile des Ausführungsbeispieles einer Anordnung nach der Erfindung. Eine Ureterschiene 1 ist an ihrer patientenseitigen Spitze mit einer Austrittsöffnung 5 und etwas unterhalb dieser Spitze mit weiteren Austrittsöffnungen 6 versehen. Ihr arztseitiges Stirnende 1' liegt in dieser bevorzugten Ausführung der Erfindung dem stirnseitigen Ende 2' einer Hilfsschiene 2 entweder nahe gegenüber oder unmittelbar daran an. Innerhalb der Hilfsschiene 2 befindet sich ein hier hohler Mandrin 3, dessen arztseitiges, verdicktes Ende mit 3' beziffert ist. In dem Lumen 14 des Mandrins 3 ist ein Steuerungsdraht bzw. Innenmandrin 4 vorgesehen, der arztseitig in einem Spritzstutzen 15 verankert ist, der mit dem verdickten Ende 3' des Mandrins 3 mittels eines Gewindes 16 verschraubbar ist.

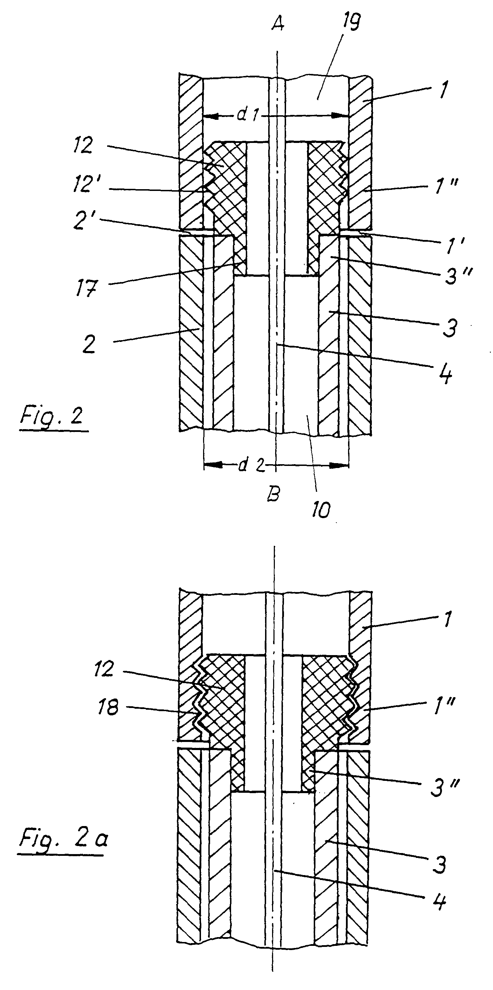

Fig. 2 zeigt im Detail eine Gewindeverbindung zwischen dem patientenseitigen Ende 3" des Mandrins 3 und dem arztseitigen Ende 1" der Ureterschiene 1. Diese Gewindeverbindung ist so gestaltet, daß sie in beiden Drehrichtungen eine Drehmitnahme zwischen Mandrin und Ureterschiene bewirkt. Mit dem Ende 3" des Mandrins 3 ist fest ein harter Gewindeteil 12 verbunden, der bevorzugt aus einem Metall wie Stahl besteht und mittels eines ringzylindrischen Teiles 17 fest mit dem Mandrinende 3" verbunden, z.B. verklebt ist. Das Gewindeteil 12 ist mit einem Außengewinde 12' versehen, das in der Ausführung gemäß Fig. 2 sich mit den Spitzen seines Gewindes in das demgegenüber wesentlich weichere und elastischere Material des Endes 1" der Ureterschiene einschraubt. Dies ist aus zeichnerischen Gründen nicht dargestellt. Statt dessen könnte auch eine Ausführung gemäß Fig. 2a vorgesehen sein, in der die zugehörige Innenseite des Ureterschienenendes 1" mit einem Innengewinde 18 versehen ist. Auch hier besteht der Gewindeteil 12 aus einem harten Material (gegebenenfalls auch einem entsprechenden harten Kunststoff) und das Ureterschienenende 1" aus einem weichen, elastischen Kunststoff. Bevorzugt ist dieses Ende 1" mit dem übrigen Bereich der Ureterschiene 1 einstückig. In beiden vorgenannten Ausführungsmöglichkeiten ist der Außendurchmesser des Gewindes 12' etwas größer als der Innendurchmesser d1 des Ureterschienenendes 1" (siehe Fig. 2) oder als der Innendurchmesser des Innengewindes 18 des Ureterschienenendes 1" (siehe Fig. 2a).

Sowohl in der Ausführung nach Fig. 2, als auch in der Ausführung nach Fig. 2a erfolgt aufgrund des nachstehend beschriebenen Verdrehens des Mandrins 3 ein Verdrehen des Gewindes 12' zur Innenwand des Ureterschienenendes 1" bzw. zu dessen Gewinde 18. Bei entsprechender Anordnung der Steigung des Gewindes hat dies ein Bewegen des Mandrins 3 mit seinem Gewindeteil 12 relativ zur Ureterschiene 1 nach unten (bezogen auf die Darstellung in der Zeichnung) zur Folge, bis sich der Gewindeteil 12 außerhalb der Ureterschiene befindet. Dabei verlaufen die Steigungen der vorgenannten Gewinde 12', 18 und der nachfolgend genannten Gewinde 21, 22 in der gleichen Schraubrichtung. Bevorzugt sind dabei die Steigungen der Gewinde einander gleich. Außerdem empfiehlt es sich, daß der Durchmesser d1 des Lumens 19 der Ureterschiene etwas kleiner ist als der Durchmesser d2 des Lumens der Hilfsschiene 2. Dies erleichtert das Eintreten des Gewindeteiles 12 in das Lumen der Hilfsschiene.

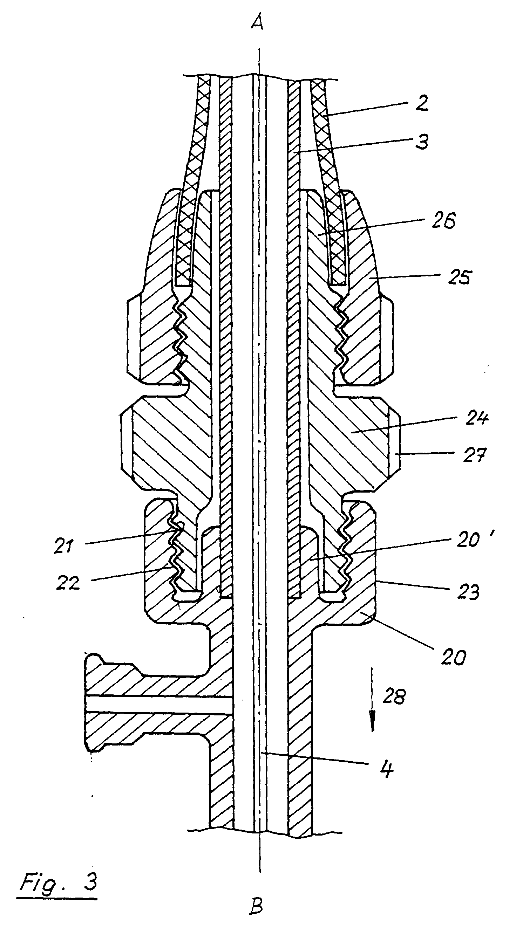

Fig. 1 zeigt schematisch ein mit dem Mandrin fest verbundenes Schraubelement 20, das ein Innengewinde 21 aufweist, das mit einem Außengewinde 22 am unteren Ende 2" der Hilfsschiene 2 in Gewindeeingriff steht. Das Schraubelement 20 ist an seiner Außenfläche 23 so ausgebildet, daß es dort zwecks Verdrehung des Mandrins 3 um seine Längsachse erfaßt werden kann. Eine bevorzugte Ausführungsform einer solchen Verbindung ist in Fig. 3 dargestellt. In diesem Ausführungsbeispiel ist das Außengewinde 22 etwa ebenso lang wie das Innengewinde 21. Das Außengewinde 22 kann aber auch nur aus wenigen Gewindegängen, im Extremfall nur aus einem Ansatz mit entsprechender Steigung bestehen, der sich an einem Halteelement 24 befindet. Das Außengewinde 22 kann auch so gestaltet sein, daß darüber ein Gewinde einer dort anzusetzenden Spritze paßt. Dieses Halteelement 24 hält die Hilfsschiene 2, die mittels einer Überwurfmutter 25 mit einem in Fig. 3 oberen und damit patientenseitigen Ansatz 26 des Halteelementes 24 lösbar verbunden und dabei in der Verbundstellung fest verklemmt ist. Das Halteelement 24 kann seitlich vorragende, flügelartige Ansätze 27 aufweisen, an denen die Bedienungsperson während des Verdrehvorganges zwischen Mandrin 3 und Hilfsschiene 2 anfassen kann. Hierzu wird mit der einen Hand die Hilfsschiene 2 oder das Halteelement 24 fest gehalten, während mit der anderen Hand das Schraubelement 20 hierzu um die Längsachse A-B der Gesamtanordnung verdreht wird. Bei entsprechender, oben bereits erwähnter Anordnung und Ausgestaltung der Steigungen der Gewinde 21, 22 bewegt sich hiermit das Schraubelement 20 in Pfeilrichtung 28 nach unten, d.h. zur Arztseite hin. Dies und die gleichzeitige Drehung des in seiner Steigung dazu passend ausgestalteten Gewindeteiles 12 zum Ureterschienenende 1" bewirkt eine Verlagerung des Mandrins 3 nach unten bei gleichzeitigem Drehen des Mandrins 3 und des Gewindeteiles 12 um die Längsachse A-B der Anordnung. Zugleich wird hiermit das Ablösen des patientenseitigen Endes des Mandrins von der Ureterschiene erreicht.

Insbesondere Fig. 3 ist zu entnehmen, daß bei dem Verdrehen der beiden Elemente 20, 24 zueinander auch ein entsprechendes Verdrehen der Hilfsschiene 2 zum Mandrin 3 erfolgt, wobei die Teile 2, 25 und 24 ein funktionell zusammenhängendes Bauteil bilden. Das gleiche gilt für die Teile 20 und 3, die über einen Stutzen 20' miteinander fest verbunden, z.B. verklebt sind.

Die Anordnung nach der Erfindung kann ab Fabrik in der in Fig. 1 dargestellten, zur Einführung in den Patientenkörper bereiten Form geliefert werden. Andernfalls werden die Teile vom Arzt nach Anweisung in die Verbindungslage gemäß Fig. 1 zusammengesetzt. Das dichte Gegenüberliegen oder Anstoßen der Stirnenden 1' der Ureterschiene und 2' der Hilfsschiene erbringt zwei Vorteile. Da die Außendurchmesser von Ureterschiene und Hilfsschiene einander gleich gewählt werden können, steht somit keine Kante des Stirnendes der Hilfsschiene oder des Stirnendes der Ureterschiene vor, die beim Einbringen sowie Hin- und Herziehen der Anordnung in den bzw. im Patientenkörper zu Verletzungen führen könnte. Außerdem bildet hierbei beim Verdrehen des Innengewindes 21 des Schraubelementes 20 zum Außengewinde 22 des Halteelementes 24 der Hilfsschiene 2 diese ein Widerlager an dem stirnseitigen Ende 1' der Ureterschiene, welches das Bewegen des Schraubelementes 20 mit Mandrin 3 in die Pfeilrichtung 28 unterstützt. Das vorstehend erwähnte Anliegen der beiden Stirnenden 1', 2' aneinander kann durch entsprechende Abstimmung der Länge der Hilfsschiene auf die Position des Schraubelementes 20 am Mandrin 3 (sofern dieser mit seinem Gewindeteil 12 in die Ureterschiene eingeschraubt ist) erreicht werden.

Befindet sich die Ureterschiene in der geforderten Position im Ureter, so ist die Spitze des Steuerungsdrahtes 4 in die Ureterschiene zurückgezogen. Diese Position wird auch durch die Befestigung bzw. Verschraubung eines solchen Steuerungsdrahtes 4 mit dem Spritzstutzen 15 gesichert, der mit dem verdickten Ende 3' des Außenmandrins verschraubt ist. Nach Lösen dieser Verschraubung kann der Innenmandrin oder Steuerungsdraht 4 aus der Ureterschiene und dem Mandrin mit Hilfe des Spritzstutzens 15 nach unten herausgezogen werden. Danach kann die Entkopplung der weiteren Teile, nämlich Außenmandrin 3 und Hilfsschiene 2 von der Ureterschiene durch Lösen der vorstehend erläuterten Schraubverbindungen 12', 1" und 21/22 erfolgen.

Die erfindungsgemäße Anordnung erlaubt ferner die Zuführung eines Röntgenkontrastmittels über das Lumen 10 des Außenmandrins 3 und das Lumen 19 der Ureterschiene 1. Das Röntgenkontrastmittel kann entweder durch die in der Spitze der Ureterschiene vorgesehene Öffnung 5 in das Nierenkelchbecken gebracht werden. Ist eine solche Öffnung nicht vorhanden, dann können hierzu auch seitliche Öffnungen 6 der Ureterschiene dienen. Dies erfolgt vor dem Entkoppeln der vorgenannten Teile der Anordnung. Die Zufuhr des Röntgenkontrastmittels kann entweder durch einen Stutzen 7 oder durch eine entsprechende Schlauchverbindung erfolgen, die an der Seite des Mandrins 3 an einer arztseitig zugängigen Stelle in das Lumen 8 dieses Mandrins hineinführt. Auch wäre die Zufuhr eines Röntgenkontrastmittels durch den arztseitigen Ausgang des Lumens 8 im Bereich der Verdickung 3' möglich. In diesem Falle müßte zuvor der Spritzstutzen 15 mit dem Steuerungsdraht 4 entfernt worden sein.

Statt des Steuerungsdrahtes 4, der in der Ausführung nach Fig. 1 dem Ausgleich der sogenannten "Memory" dient, kann man auch einen entsprechend dünnen Innenmandrin vorsehen, wobei sich aber der Außenmandrin 3 in seiner Ausgestaltung und Funktion nicht ändert. Ein solcher dünner Innenmandrin kann eine weiche Spitze haben, so daß er auch bei Herausragen aus der Öffnung 5 der Ureterschiene den Patientenkörper nicht verletzt. In einer anderen Ausführung eines Innenmandrins kann dieser so stabil sein, daß er eine der Ureterschienenspitze ab Fabrik eingegebene, bleibende Krümmung (sog. "Memory") ausgleichen kann.

Die Erfindung erlaubt ferner die Verwendung eines Doppelmandrins, bei dem der Innenmandrin eine versteifte, die vorgenannte "Memory" bildende Spitze aufweist. Der Außenmandrin ist auch in diesem Fall gemäß der Erfindung ausgestaltet.

Soll ein Steuerungsdraht oder ein Innenmandrin aus der Ureterschienenspitze herausgeschoben, so muß hierzu die schon erläuterte Öffnung 5 vorgesehen sein. Auch ist eine Anordnung einsetzbar, bei der der Innenmandrin ein relativ steifer, hohler Steuerungsdraht ist, in dem sich ein dünner, weicher und dazu in Längsrichtung verschiebbarer weiterer Steuerungsdraht befindet. Auch kann ein hohler Steuerungsdraht vorgesehen sein, der am patientenseitigen Ende eine "Memory" aufweist und in dessen Innern sich ein Versteifungsdraht befindet.

## Patentansprüche

1. Anordnung mit einer Ureterschiene (1), einem Mandrin (3) und einer Hilfsschiene (2), wobei eine lösbare Gewindeverbindung zwischen dem Mandrin und der hohlen Ureterschiene vorgesehen ist, welche die Ureterschiene auf Drehmitnahme mit dem Mandrin verbindet, wobei nach Lösen dieser Gewindeverbindung der Mandrin in Längsrichtung der Ureterschiene zu dieser verschiebbar, erforderlichenfalls ganz herausziehbar ist, und wobei der Mandrin mittels einer von Hand zu betätigenden Vorrichtung zur Ureterschiene und Hilfsschiene um die gemeinsame Längsachse verdrehbar ist, **dadurch gekennzeichnet, daß** der Mandrin (3) mit der Hilfsschiene (2) in einer Gewindeverbindung steht, die eine Drehmitnahme in beiden Drehrichtungen zwischen diesen beiden Teilen (2, 3) bildet und sich bei in den Ureter eingeführter Ureterschiene (1) außerhalb des Körpers des Patienten befindet.

2. Anordnung nach Anspruch 1, **dadurch gekennzeichnet, daß** die lösbare Gewindeverbindung zwischen dem Mandrin (3) und der Ureterschiene (1) zur Drehmitnahme in beiden Drehrichtungen ausgebildet ist.

3. Anordnung nach Anspruch 1 oder 2, **dadurch gekennzeichnet, daß** die patientenseitige Spitze (12) des Mandrin ein Außengewinde (12') aus einem harten Material aufweist und damit sich im Innern des arztseitigen Endes (1") der Ureterschiene (3) befindet oder darin einbringbar ist, das aus einem demgegenüber weicheren und elastischeren Material besteht, wobei der Außendurchmesser des Außengewindes (12') der Mandrinspitze (12) etwas größer ist als der Innendurchmesser des daran anliegenden Bereiches der Ureterschiene.

4. Anordnung nach einem der Ansprüche 1 bis 3, **dadurch gekennzeichnet, daß** die Gewindeverbindung zwischen Mandrin (3) und Hilfsschiene (2) aus zwei ineinander greifenden Gewinden (21, 22) besteht, wobei die Hilfsschiene und der Mandrin um ihre Längsrichtung (A-B) relativ zueinander verdrehbar sind.

5. Anordnung nach Anspruch 4, **dadurch gekennzeichnet, daß** das Gewinde (21) des Mandrins (3) sich an einem Schraubelement (20) befindet, das außenseitig für das Erfassen von Hand ausgebildet bzw. geeignet ist und innenseitig das Gewinde (21) aufweist, wobei das Schraubelement (20) fest mit dem Mandrin verbunden ist.

6. Anordnung nach Anspruch 5, **dadurch gekennzeichnet, daß** das Schraubelement mit einer Innenbohrung und einem dazugehörigen hohlen Stutzen (20') mit der Außenseite des Mandrins (3) verklebt ist.

7. Anordnung nach einem der Ansprüche 4 bis 6, **dadurch gekennzeichnet, daß** das arztseitige Ende der Hilfsschiene an einem Halteelement (24) befestigt ist, dessen Gewinde (22) in Eingriff mit dem Gewinde (21) des Schraubelementes (20) ist und das zusammen mit der Hilfsschiene (2) um die Längsachse (A-B) relativ zum Mandrin (3) verdrehbar ist.

8. Anordnung nach einem der Ansprüche 5 bis 7, **dadurch gekennzeichnet, daß** das Gewinde (21) des Schraubelementes (20) als Innengewinde und das Gewinde (22) des Halteelementes (24) als damit im Eingriff stehendes Außengewinde ausgebildet ist, und daß das Außengewinde (22) sich entweder über die gesamte Länge des Innengewindes (21) oder nur über einen Teilbereich erstreckt.

9. Anordnung nach Anspruch 8, **dadurch gekennzeichnet, daß** das Außengewinde (22) des Halteelementes (24) nur aus einigen Gewindegängen besteht, die sich von der Stirnfläche des Halteelementes her erstrecken und bevorzugt so ausgestaltet sind, daß darüber das Gewinde einer am Halteelement zu befestigenden Spritze paßt.

10. Anordnung nach einem der Ansprüche 5 bis 9, **dadurch gekennzeichnet, daß** das Halteelement (24) mit nach außen ragenden Handhaben, z.B. in Form von Flügeln (27), versehen ist.

11. Anordnung nach einem der Ansprüche 5 bis 10, **dadurch gekennzeichnet, daß** das arztseitige Ende der Hilfsschiene (2) mittels einer Überwurfmutter (25) mit dem Halteelement (24) fest aber lösbar verbunden ist.

12. Anordnung nach einem der Ansprüche 1 bis 11, **dadurch gekennzeichnet, daß** die Länge der Hilfsschiene (2) und die Position ihrer Gewindeverbindung (21/22) mit dem Mandrin (3) so aufeinander abgestimmt sind, daß bei einsatzbereiter Anordnung sich die arztseitige Stirnkante (1') der Ureterschiene (1) und die patientenseitige Stirnkante (2') der Hilfsschiene (2) aneinander dicht gegenüberliegen oder aneinander anliegen.

13. Anordnung nach einem der Ansprüche 1 bis 12, **dadurch gekennzeichnet, daß** der Gewindeteil (12, 12') an der Spitze des Mandrins (3) ein gesonderter Bauteil (12) mit Außengewinde (12') ist, der mit dem patientenseitigen Ende (3") des Mandrins (3) fest verbunden ist.

14. Anordnung nach Anspruch 13, **dadurch gekennzeichnet, daß** der Gewindeteil (12) aus einem harten Metall, z.B. Stahl besteht.

15. Anordnung nach einem der Ansprüche 1 bis 14, **dadurch gekennzeichnet, daß** die Innenseite des arztseitigen Endes (1") der Ureterschiene (1) ein Innengewinde (18) aufweist, das zum Außengewinde (12') der Mandrinspitze paßt.

16. Anordnung nach einem der Ansprüche 1 bis 15, **dadurch gekennzeichnet, daß** die jeweiligen Gewinde (12, 12'; 18; 21, 22) die gleiche Schraubrichtung und die gleiche Steigung aufweisen.

17. Anordnung nach Anspruch 15 oder 16, **dadurch gekennzeichnet, daß** das Innengewinde (18) des arztseitigen Endes (1") der Ureterschiene (1) sich an einem gesonderten Teil befindet, der an der Innenseite des arztseitigen Endes fest angebracht ist.

## Claims

1. Assembly with a ureter rail (1), a mandrin (3) and an auxiliary rail (2), wherein between the mandrin and the hollow ureter rail is provided a releasable threaded joint which connects the ureter rail to the mandrin for rotational entrainment, wherein after release of this threaded joint the mandrin is displaceable in the longitudinal direction of the ureter rail relative to the latter, if necessary can be pulled out completely, and wherein the mandrin is rotatable about the common longitudinal axis relative to the ureter rail and auxiliary rail by means of a manually operated device, **characterised in that** the mandrin (3) is connected to the auxiliary rail (2) in a threaded joint which forms a rotational entrainment means in both directions of rotation between these two parts (2, 3) and is located outside the body of the patient when the ureter rail (1) is introduced into the ureter.

2. Assembly according to claim 1, **characterised in that** the releasable threaded joint between the mandrin (3) and the ureter rail (1) is designed for rotational entrainment in both directions of rotation.

3. Assembly according to claim 1 or 2, **characterised in that** the tip (12) of the mandrin on the patient's side comprises an external thread (12') made of a hard material and hence is located in or can be introduced into the interior of the end (1") of the ureter rail (3) on the doctor's side, which thread is made of a material which is softer and more resilient by comparison, wherein the outside diameter of the external thread (12') of the mandrin tip (12) is slightly larger than the inside diameter of the region of the ureter rail in contact therewith.

4. Assembly according to any of claims 1 to 3, **characterised in that** the threaded joint between mandrin (3) and auxiliary rail (2) consists of two interlocking threads (21, 22), wherein the auxiliary rail and the mandrin are rotatable relative to each other about their longitudinal direction (A-B).

5. Assembly according to claim 4, **characterised in that** the thread (21) of the mandrin (3) is located on a screw element (20) which on the outside is designed or suitable for gripping by hand and on the inside comprises the thread (21), wherein the screw element (20) is rigidly connected to the mandrin.

6. Assembly according to claim 5, **characterised in that** the screw element is adhered by an internal bore and an associated hollow connection piece (20') to the outside of the mandrin (3).

7. Assembly according to any of claims 4 to 6, **characterised in that** the end of the auxiliary rail on the doctor's side is attached to a holding element (24) whose thread (22) is engaged with the thread (21) of the screw element (20) and which together with the auxiliary rail (2) is rotatable relative to the mandrin (3) about the longitudinal axis (A-B).

8. Assembly according to any of claims 5 to 7, **characterised in that** the thread (21) of the screw element (20) is constructed as an internal thread and the thread (22) of the holding element (24) is constructed as an external thread engaged therewith, and **in that** the external thread (22) extends either over the whole length of the internal thread (21) or over only a part.

9. Assembly according to claim 8, **characterised in that** the external thread (22) of the holding element (24) consists of only a few thread turns which extend from the end face of the holding element and are preferably designed in such a way that the thread of a syringe to be attached to the holding element fits over them.

10. Assembly according to any of claims 5 to 9, **characterised in that** the holding element (24) is provided with outwardly extending handles, e.g. in the form of wings (27).

11. Assembly according to any of claims 5 to 10, **characterised in that** the end of the auxiliary rail (2) on the doctor's side is rigidly but releasably connected to the holding element (24) by means of a union nut (25).

12. Assembly according to any of claims 1 to 11, **characterised in that** the length of the auxiliary rail (2) and the position of its threaded joint (21/22) with the mandrin (3) are adapted to each other in such a way that, when the assembly is ready for use, the end edge (1') of the ureter rail (1) on the doctor's side and the end edge (2') of the auxiliary rail (2) on the patient's side are located tightly opposite each other or abut against each other.

13. Assembly according to any of claims 1 to 12, **characterised in that** the threaded portion (12, 12') at the tip of the mandrin (3) is a separate component (12) with external thread (12') which is rigidly connected to the end (3") of the mandrin (3) on the patient's side.

14. Assembly according to claim 13, **characterised in that** the threaded portion (12) is made of a hard metal, e.g. steel.

15. Assembly according to any of claims 1 to 14, **characterised in that** the inside of the end (1") of the ureter rail (1) on the doctor's side comprises an internal thread (18) which fits the external thread (12') of the mandrin tip.

16. Assembly according to any of claims 1 to 15, **characterised in that** the respective threads (12, 12'; 18; 21, 22) have the same screwing direction and the same pitch.

17. Assembly according to claim 15 or 16, **characterised in that** the internal thread (18) of the end (1") of the ureter rail (1) on the doctor's side is located on a separate part which is rigidly mounted on the inside of the end on the doctor's side.

## Revendications

1. Agencement avec un guide urétéral (1), un mandrin (3) et un guide auxiliaire (2), dans lequel est prévue une liaison filetée libérable entre le guide urétéral creux et le mandrin, qui relie le guide urétéral au mandrin par un entraînement à rotation, le mandrin pouvant, après libération de cette liaison filetée, coulisser dans le sens longitudinal par rapport au guide urétéral ou en être complètement extrait, et le mandrin pouvant tourner autour de l'axe longitudinal commun, par rapport au guide urétéral et au guide auxiliaire, à l'aide d'un dispositif à actionner à la main, **caractérisé en ce que** le mandrin (3) est relié au guide auxiliaire (2) par un filetage qui forme un entraînement à rotation dans les deux sens de rotation, entre ces deux pièces (2, 3), et qui se situe à l'extérieur du corps du patient lorsque le guide urétéral (1)est inséré dans l'urètre.

2. Agencement selon la revendication 1, **caractérisé en ce que** la liaison filetée libérable entre le mandrin (3) et le guide urétéral (1), est configurée pour permettre un entraînement en rotation dans les deux sens de rotation.

3. Agencement selon la revendication 1 ou 2, **caractérisé en ce que** la pointe (12) du mandrin située du côté du patient présente un filet extérieur (12') en matériau dur, et se trouve ainsi à l'intérieur de l'extrémité (1") du guide urétéral (3) située du côté du médecin, ou peut y être insérée, cette extrémité étant constituée d'un matériau plus malléable et plus élastique, le diamètre extérieur du filet extérieur (12') de la pointe (12) du mandrin étant légèrement plus grand que le diamètre intérieur de la partie du guide urétéral qui lui est contiguë.

4. Agencement selon l'une des revendications 1 à 3, **caractérisé en ce que** la liaison filetée entre le mandrin (3) et le guide auxiliaire (2) est constituée de deux filets (21, 22) qui s'engagent l'un dans l'autre, le guide auxiliaire et le mandrin pouvant être tournés l'un par rapport à l'autre autour de leur direction longitudinale (A - B).

5. Agencement selon la revendication 4, **caractérisé en ce que** le filet (21) du mandrin (3) est situé sur un élément à visser (20) dont le côté extérieur est configuré de manière à pouvoir être saisi à la main ou convient à ce but, et dont le côté intérieur présente le filet (21), l'élément à visser (20) étant solidaire du mandrin.

6. Agencement selon la revendication 5, **caractérisé en ce que** l'élément à visser, qui présente un alésage intérieur et une tubulure creuse (20') qui y est associée, est collé sur le côté extérieur du mandrin (3).

7. Agencement selon l'une des revendications 4 à 6, **caractérisé en ce que** l'extrémité du guide auxiliaire située du côté du médecin est fixée à un élément de retenue (24) dont le filet (22) s'engage sur le filet (21) de l'élément à visser (20) et qui, avec le guide auxiliaire (2), peut tourner par rapport au mandrin (3) autour de l'axe longitudinal (A - B).

8. Agencement selon l'une des revendications 5 à 7, **caractérisé en ce que** le filet (21) de l'élément à visser (20) est configuré comme filet intérieur, et le filet (22) de l'élément de retenue (24) comme filet extérieur en prise avec celui-ci, et **en ce que** le filet extérieur (22) s'étend sur toute la longueur du filet intérieur (21) ou seulement sur une partie de ce dernier.

9. Agencement selon la revendication 8, **caractérisé en ce que** le filet extérieur (22) de l'élément de retenue (24) n'est constitué que de quelques pas de filet qui s'étendent depuis la surface frontale de l'élément de retenue et qui sont de préférence configurés de telle sorte que le filet reçoit par leur intermédiaire une seringue à fixer sur l'élément de retenue.

10. Agencement selon l'une des revendications 5 à 9, **caractérisé en ce que** l'élément de retenue (24) est doté de poignées débordant vers l'extérieur, qui présentent par exemple la forme d'ailettes (27).

11. Agencement selon l'une des revendications 5 à 10, **caractérisé en ce que** l'extrémité du guide auxiliaire (2) qui est située du côté du médecin est reliée de manière solidaire mais libérable à l'élément de retenue (24) par l'intermédiaire d'un écrou d'accouplement (25).

12. Agencement selon l'une des revendications 1 à 11, **caractérisé en ce que** la longueur du guide auxiliaire (2) et la position de sa liaison filetée (21/22) avec le mandrin (3) sont accordées l'une à l'autre de telle sorte que, lorsque l'agencement est prêt à être utilisé, le bord frontal (1'), situé du côté du médecin, du guide urétéral (1) et le bord frontal (2'), situé du côté du patient, du guide auxiliaire (2) sont disposés tout près l'un de l'autre, ou reposent l'un contre l'autre.

13. Agencement selon l'une des revendications 1 à 12, **caractérisé en ce que** la partie filetée (12, 12') située à la pointe du mandrin (3) est un composant (12) distinct doté d'un filet extérieur (12') et fixé solidairement à l'extrémité (3") du mandrin (3) qui est située du côté du patient.

14. Agencement selon la revendication 13,
**caractérisé en ce que** la partie filetée (12) est constituée d'un métal dur, par exemple de l'acier.

15. Agencement selon l'une des revendications 1 à 14, **caractérisé en ce que** le côté intérieur de l'extrémité (1") du guide urétéral (1) qui est située du côté du médecin présente un filet intérieur (18) qui s'adapte au filet extérieur (12') de la pointe du mandrin.

16. Agencement selon l'une des revendications 1 à 15, **caractérisé en ce que** chaque filet (12, 12' ; 18; 21, 22) présente le même sens de vissage et le même pas.

17. Agencement selon la revendication 15 ou 16, **caractérisé en ce que** le filet intérieur (18) de l'extrémité (1") du guide urétéral (1) qui est située du côté du médecin se trouve sur une pièce distincte qui est fixée au côté intérieur de l'extrémité située du côté du médecin.
